# EUROPEAN PATENT APPLICATION

(11) **EP 4 767 970 A1**
(43) Date of publication of application: **01.07.2026**
(21) Application number: 25221012.5
(22) Date of filing: 05.12.2025
(51) Int. Cl.: A61B 18/14, A61B 5/287

(54) **PLANAR CATHETER WITH A FLEXIBLE CIRCUIT INCLUDING A REINFORCED PORTION**

(30) Priority: 31.12.2024 US 202419007322
(71) Applicant: Biosense Webster (Israel) Ltd., 2066717 Yokneam (IL)
(72) Inventor: EBRAHIMI, Babak, Irvine, 92618 (US); ABBAS, Mohammad, Irvine, 92618 (US); RODRIGUEZ SOTO, Juan, Irvine, 92618 (US); VAN NIEKERK, Pieter Emmelius, Irvine, 92618 (US); ROUSU, Corey M., Irvine, 92618 (US)
(74) Representative: Carpmaels & Ransford LLP

(57) **Abstract**

The disclosed technology includes a flexible circuit for an end effector of a medical probe. The flexible circuit comprises a flexible substrate layer, a plurality of electrodes, and a stiffening layer. The stiffening layer is disposed on the first side of the flexible substrate layer and in the first zone of the flexible circuit such that a first thickness of a first zone of the flexible circuit is greater than a second thickness of the second zone of the flexible circuit, the thickness being measured along the vertical axis, and such that a first flexural rigidity of the first zone is greater than a second flexural rigidity of a second zone of the flexible substrate layer.

## Description

### FIELD

The present technology relates generally to medical devices, and in particular medical probes with electrodes, and further relates to, but not exclusively, medical probes suitable for use to map and/or ablate tissue.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to conform closely to the target anatomy. For mapping within an atria or a ventricle (for example, an apex of a ventricle), it is desirable for a catheter to collect larger amounts of data signals within shorter time spans. It is also desirable for such a catheter to be capable of allowing sufficient electrode contact with different tissue surfaces, for example, flat, curved, irregular or nonplanar surface tissue, and be collapsible for atraumatic advancement and withdrawal through a patient's vasculature. Existing catheters generally require stiff internal structural members to ensure that a predetermined configuration is maintained. The stiffness is a disadvantage during manipulation in the body organ as it can prevent electrodes from contacting the tissue.

Other catheters can include flexible end effectors designed to overcome this disadvantage. These catheters can include layered components that can be time-consuming, complex, and expensive to manufacture and assemble. Moreover, electrical traces and other components associated therewith can be prone to breakage and/or delamination when in use.

### SUMMARY

There is provided, in accordance with the disclosed technology, a flexible circuit for an end effector of a medical probe. The flexible circuit comprises a flexible substrate layer, a plurality of electrodes, and a stiffening layer. The flexible substrate layer extends along a longitudinal axis and comprises a first side and a second side along a vertical axis that is orthogonal to the longitudinal axis, a plurality of tines extending along the longitudinal axis, a first zone comprising a portion of at least one of the tines of the plurality of tines, and a second zone comprising another portion of the plurality of tines. The plurality of electrodes is disposed on each of the plurality of tines of the flexible substrate layer. The stiffening layer is disposed on the first side of the flexible substrate layer and in the first zone of the flexible circuit such that (i) a first thickness of the first zone of the flexible circuit is greater than a second thickness of the second zone of the flexible circuit, the thickness being measured along the vertical axis, and (ii) a first flexural rigidity of the first zone is greater than a second flexural rigidity of the second zone.

There is further provided, in accordance with the disclosed technology, an end effector for a medical probe. The end effector comprises an insulative material, a framework, a first flexible circuit, a plurality of electrodes, and a stiffening layer. The framework is disposed in the insulative material, with the framework being approximately planar along a first longitudinal axis. The first flexible circuit is disposed in the insulative material such that the first flexible circuit is spaced apart from the framework along a vertical axis that is orthogonal to the first longitudinal axis. The first flexible circuit comprises a first flexible substrate layer extending along a second longitudinal axis parallel to the first longitudinal axis. The first flexible substrate layer comprises a first side and a second side along a vertical axis that is orthogonal to the longitudinal axis, a plurality of tines extending along the longitudinal axis, a first zone comprising a portion of at least one of the tines of the plurality of tines, and a second zone comprising another portion of the plurality of tines. The plurality of electrodes is disposed on each of the plurality of tines of the flexible substrate layer. The stiffening layer is disposed on the first side of the flexible substrate layer and in the first zone of the flexible circuit such that (i) a first thickness of the first zone of the flexible circuit is greater than a second thickness of the second zone of the flexible circuit, the thickness being measured along the vertical axis, and (ii) a first flexural rigidity of the first zone is greater than a second flexural rigidity of the second zone.

There is further provided, in accordance with the disclosed technology, an end effector for a medical probe. The end effector comprises an insulative material, a framework, a first flexible circuit, a plurality of electrodes, and a stiffening layer. The framework is disposed in the insulative material, with the framework being approximately planar along a first longitudinal axis. The first flexible circuit is disposed in the insulative material. The first flexible circuit comprises a first flexible substrate layer extending along a second longitudinal axis parallel to the first longitudinal axis. The first flexible substrate layer comprises a first side and a second side along a vertical axis that is orthogonal to the longitudinal axis, a plurality of tines extending along the longitudinal axis, a first zone comprising a portion of at least one of the tines of the plurality of tines, and a second zone comprising another portion of the plurality of tines. The plurality of electrodes is disposed on each of the plurality of tines of the flexible substrate layer. The stiffening layer is disposed on the insulative material on the first side of the flexible substrate layer and in the first zone of the flexible circuit such that (i) a first thickness of the first zone of the flexible circuit is greater than a second thickness of the second zone of the flexible circuit, the thickness being measured along the vertical axis, and (ii) a first flexural rigidity of the first zone is greater than a second flexural rigidity of the second zone.

There is further provided, in accordance with the disclosed technology, a method. The method comprises forming a flexible substrate layer of a flexible circuit. The flexible substrate layer comprises a first surface disposed on a first side of the flexible substrate layer, a second surface disposed on a second side of the flexible substrate layer, a plurality of tines, a first zone comprising a portion of at least one of the tines of the plurality of tines, and a second zone comprising another portion of the plurality of tines. The method comprises forming a plurality of electrodes on the first surface. The method comprises forming a stiffening layer on the first side of the flexible substrate layer and in the first zone of the flexible circuit such that (i) a first thickness of the first zone of the flexible circuit is greater than a second thickness of the second zone of the flexible circuit, the thickness being measured along a vertical axis, and (ii) a first flexural rigidity of the first zone is greater than a second flexural rigidity of the second zone.

### BRIEF DESCRIPTION OF THE DRAWINGS

FIG. 1 is a schematic pictorial illustration of a medical system including a medical device that includes a medical probe with an end effector with electrodes, in accordance with the disclosed technology;
FIG. 2 is a schematic pictorial illustration showing an exploded perspective view of an exemplary end effector, in accordance with the disclosed technology;
FIG. 3 is a schematic pictorial illustration showing a plan view of a flexible circuit including a first stiffening layer, in accordance with the disclosed technology;
FIG. 4A is a schematic pictorial illustration showing a cross-sectional view of a portion of the flexible circuit and the first stiffening layer, taken along line 4A-4A in FIG. 3, in accordance with the disclosed technology;
FIG. 4B is a schematic pictorial illustration showing a detail view of Detail A in FIG. 4A, in accordance with the disclosed technology;
FIG. 5A is a schematic pictorial illustration showing a plan view of the flexible circuit including a second stiffening layer, in accordance with the disclosed technology;
FIG. 5B is a schematic pictorial illustration showing a cross-sectional view of the flexible circuit and the stiffening layer of FIG. 5A, taken along line 5B-5B in FIG. 5A, in accordance with the disclosed technology;
FIG. 5C is a schematic pictorial illustration showing a cross-sectional view, similar to that of FIG. 5B, of the flexible circuit and an alternative stiffening layer configuration, in accordance with the disclosed technology;
FIG. 6A is a schematic pictorial illustration showing a detail view of a portion of the flexible circuit of FIG. 3, provided with a modified first or second stiffening layer, in accordance with the disclosed technology;
FIG. 6B is a schematic pictorial illustration showing a detail view of a portion of the flexible circuit of FIG. 3, provided with an alternative modified first or second stiffening layer, in accordance with the disclosed technology;
FIG. 7 is a schematic pictorial illustration showing a medical probe assembly, in accordance with the disclosed technology;
FIG. 8 is a flow chart of a manufacturing method, in accordance with the disclosed technology;
FIG. 9 is a schematic pictorial illustration showing an exploded perspective view of another exemplary end effector, in accordance with the disclosed technology; and
FIG. 10 is a schematic pictorial illustration showing an exploded perspective view of the exemplary end effector of FIG. 9 including a stiffening layer, in accordance with the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" or "generally" for any numerical values or ranges indicate a suitable dimensional tolerance that allows the part or collection of components to function for its intended purpose as described herein. More specifically, "about" or "approximately" or "generally" may refer to the range of values ±20% of the recited value, e.g. "about 90%" may refer to the range of values from 71% to 110%. For further example, "generally parallel" may refer to the range of values of parallel (i.e., 0 degree angle relative to one another) ±20 degrees. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

The present disclosure is related to systems, methods, uses, and devices for mapping and ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

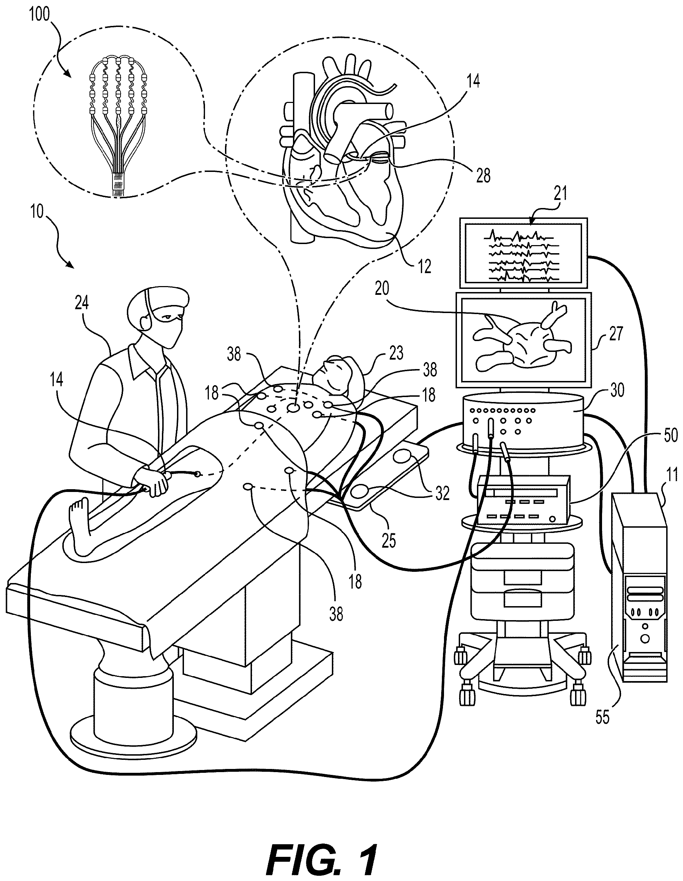

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter/medical probe 14 that is configured for sensing IEGM is illustrated herein. Physician 24 brings a catheter shaft with a distal tip 28 (*e.g.,* a multi-layered end effector 100) of catheter 14 into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 102 optionally distributed over end distal tip 28 coupled to a catheter shaft and configured to sense the IEGM signals as described in more detail below. Catheter 14 may additionally include a position sensor embedded in or near end distal tip 28 for tracking position and orientation of distal tip 28. Optionally and preferably, position sensor is a magnetic based position sensor including multiple magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of end effector 100 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 102. For impedance-based tracking, electrical current is directed toward electrodes 102 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 102 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 160A, 160B at an end effector of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618.

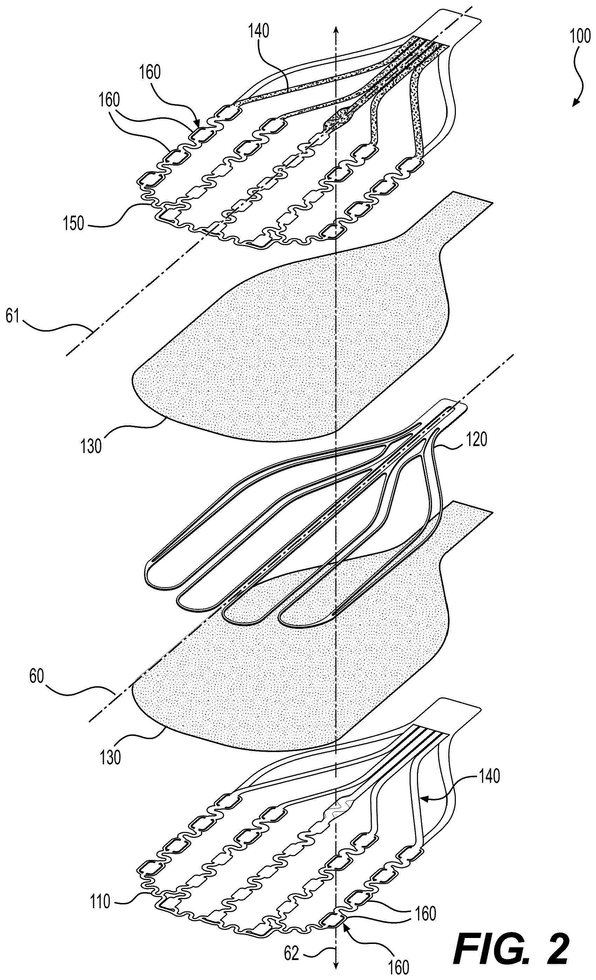

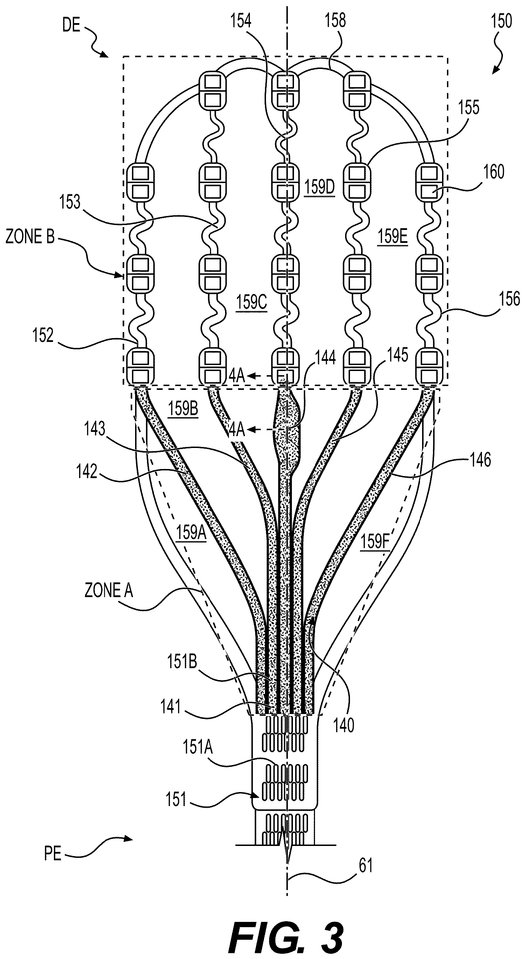

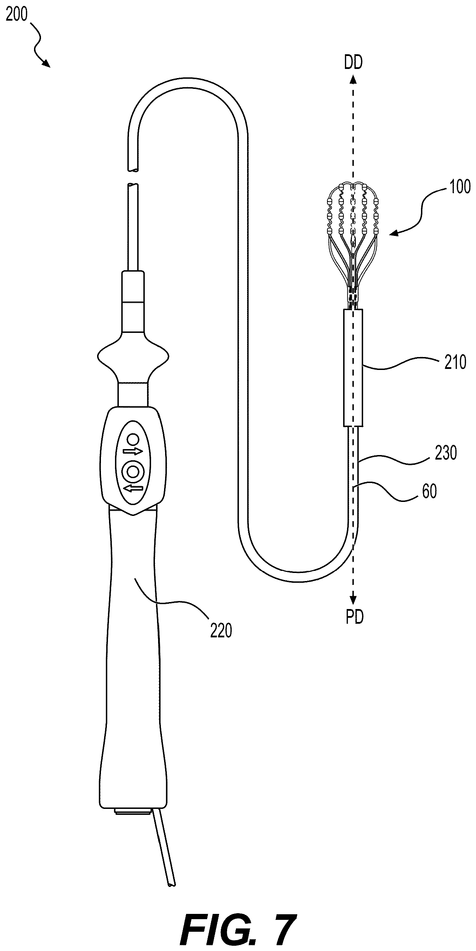

FIGs. 2-4B provide various views of one or more portions of an end effector 100 (the term "end effector" is used synonymously with the term "distal tip" herein) that is configured for insertion into an internal body cavity of a patient. In particular, these figures depict an end effector 100 that includes a stiffening layer 140 (denoted by stippling in FIGs. 2 and 3) that reduces the stress on electrical interconnections disposed at a proximal section thereof. The stiffening layer 140 aids in reducing kinks in the end effector as the end effector bends and deflects during use and as it collapses into a sheath 210 (FIG. 7) and increases the bend radius in the proximal section. The following disclosure will elucidate additional benefits of the described configurations to those skilled in the art.

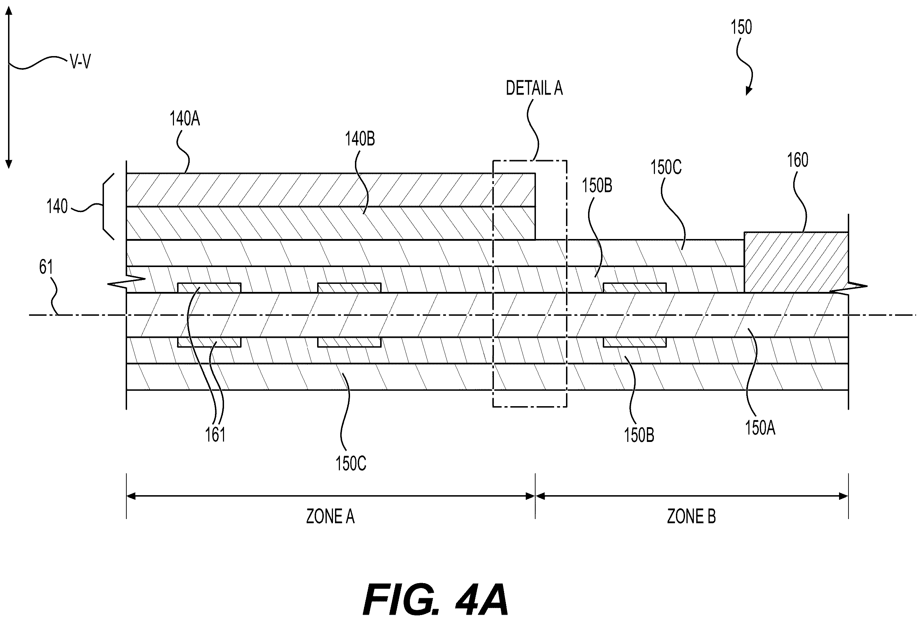

Specifically, FIG. 2 shows an exploded view of the first end effector 100, with the components thereof extending along a longitudinal axis 60 of the end effector 100 and exploded vertically along a vertical axis 62, FIG. 3 show a plan view of a flexible circuit 150 that is reinforced with a stiffening layer 140, and FIGs. 4A-4B are detail cross-sectional views of the flexible circuit 150. An opposing flexible circuit 110 is similarly or identically designed in this example. Therefore, it is appreciated that any description of the first flexible circuit 150 also characterizes the configuration of the second flexible circuit 110, and vice versa, unless explicitly noted to the contrary.

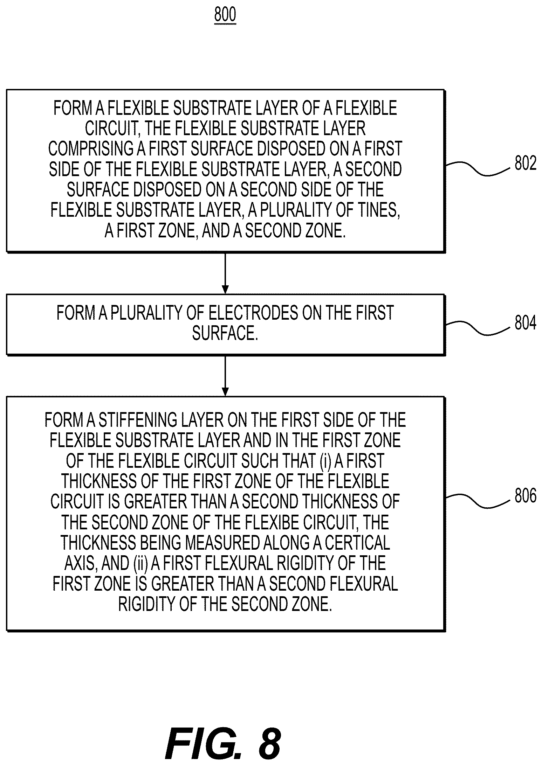

The end effector 100 extends from a proximal end (upper right-hand side of FIG. 2), that connects to an elongated shaft 230 (FIG. 8), to a distal end (bottom left-hand side of FIG. 2) along a longitudinal axis 60, and includes components contiguous with and/or disposed within an insulative material 130. A first flexible circuit 150 (and the second flexible circuit 110) of the end effector 100 extends along another longitudinal axis 61 parallel to the overall end effector longitudinal axis 60. The first flexible circuit 150 and second flexible circuit 110 additionally include a plurality of electrodes 160 electrically connected to the PIU 30 via electrical interconnections 161 (e.g., electrical traces, see FIG. 4A).

In some examples, the term "flexible circuit" includes thin-film circuit, flexible printed circuit board, thin film deposition via lithography and etching processes on substrates such as polyimide, copper, LCP, nitinol substrate, thermoplastic polyurethane (TPU), silicone, thermoset resin, or other polymeric substrates. In some examples, the flexible circuits described herein can be made primarily of polyimide. In other examples, it can be made of any of biocompatible polyimides, glass-reinforced epoxy laminate materials, copper, or graphene, alone or in combination. In some examples, the electrodes 160 described herein can include at least one mapping electrode and/or at least one ablation electrode and can be configured to detect electrophysiological signals or transmit ablative energy AC or DC from an energy generator 50 to the tissue according to the various ablation methods previously described e.g., RF, IRE, etc.

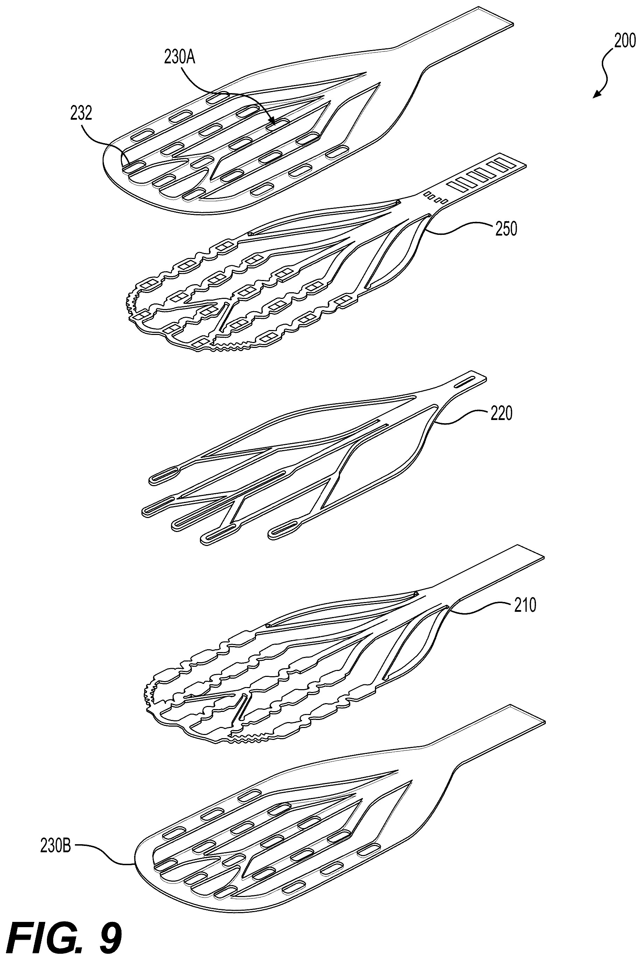

The end effector 100 can further include a framework 120 contiguous to the insulative material 130 or in the insulative material 130. In examples in which the distal tip 28 includes framework 120, the framework 120 can be disposed directly on the first flexible circuit 150 (or both the first flexible circuit 150 and the second flexible circuit 110) with none, or very little, of the insulative material 130 coming between the two. An example of this is described with respect to FIGs. 9-10. In other words, the two longitudinal axes 60, 61 previously referenced can be disposed proximal to one another. In other examples, insulative layers of the insulative material 130 can space the framework 120 from the flexible circuits 110, 150 such that the longitudinal axes 60, 61 are spaced further than the previously described exemplary configuration. In some examples, the framework 120 is disposed in the insulative material 130 and is substantially planar along the longitudinal axis 60 such that the longitudinal axis 60 is parallel to or coincident with the framework 120. In some examples, the framework 120 is symmetric relative to the longitudinal axis 60. In some examples, the framework 120 is formed from a flexible, resilient material. By way of example, the framework can be formed from a shape-memory alloy such as nickel-titanium, also known as Nitinol, cobalt chromium, stainless steel, and/or other alloys that exhibit pseudo-elastic and/or super-elastic properties. The framework 120 can be formed from a planar or cylindrical stock of material using any suitable method. For example, the framework 120 can be formed by cutting, laser cutting, stamping, etc.

While not explicitly illustrated, it is noted that the layered end effector 100 shown in FIG. 2 can also include other layers, such as a location sensing loop layer for sensing a position and/or shape of the end effector 100.

As discussed above, the flexible circuits 110, 150 are disposed in an insulative material 130 that extends along the longitudinal axis 60. The insulative material 130 can be contiguous to the contact surfaces of the electrodes 160 so that only the contact surfaces of at least a portion of the plurality of electrodes 160 are exposed to the ambient environment. As used herein, "ambient environment" refers to the external environment such as the organ in which the first end effector 100 is deployed or in the operating theater prior to being deployed in the biological organ. The insulative material 130 at least partially encapsulates and/or spaces the different layers of the end effector 100 (e.g., the flexible circuits 110, 150 and the framework 120) along the vertical axis 62.

In the present example, all of the electrodes 160 are exposed through the insulative material. It is noted that not all of the electrodes 160 on the flexible circuits 110, 150 necessarily need be exposed through the insulative material 130 as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood.

Insulative material 130 can include one or more sheets fused together proximate the framework 120 into a single, contiguous, generally planar insulative mass 130. This insulative material 130 also serves to enhance the atraumaticity of the end effector tip 100 and to protect the subject from sharp edges. The insulative material 130 can include polymer. The insulative material 130 can be heat formed around at least a portion of the first flexible circuit 150, the second flexible circuit 110, and the framework 120. The polymer can include TPU or other heat formed or shaped material which lends itself to said heat forming. In some examples, the insulative material 130 has a Shore A hardness of approximately 52 (e.g., a Shore A hardness in the range of 50-55).

Furthermore, while the insulative material 130 is shown to be flat in these figures, insulative material 130 can be shaped, scalloped, ribbed, ridged, concaved, convexed, or otherwise configured such that the overall profile of insulative material 130 yields physical and/or mechanical properties, such as rigidity and flexion along multiple axes, required by the distal tip 28/end effector 100, mentioned above.

Making specific reference to FIGs. 3 and 4A in conjunction with one another, the flexible circuit 150 includes a flexible substrate layer 150A and a coverlay 150C connected on both sides to the flexible substrate layer 150A via, for example, an adhesive material layer 150B. As seen in FIG. 4A, the flexible substrate layer 150A has a first side (e.g., upper side seen in FIG. 4A) and a second side (e.g., lower side shown in FIG. 4A) along a vertical direction V-V (which is co-axial with the vertical axis 61 and orthogonal to the longitudinal axis 61) of the end effector 100. While the flexible circuit 150 is detailed in the following description, it is again noted that all of the following details can or are also applied to the second flexible circuit 110 unless explicitly noted to the contrary.

The flexible substrate layer 150A of the flexible circuit 150 extends along the longitudinal axis 61 of the flexible circuit 150 comprises a bio-compatible material. In some examples, the flexible substrate layer 150A is formed entirely from or about entirely from the bio-compatible material. In some examples, the flexible substrate layer is formed from polyimide, copper, LCP, nitinol substrate, thermoplastic polyurethane (TPU), silicone, thermoset resin, or other polymeric substrates. In some examples, the flexible substrate layer 150A described herein can be made primarily of polyimide. In other examples, it can be made of any of biocompatible polyimides, glass-reinforced epoxy laminate materials, copper, or graphene, alone or in combination.

The electrodes 160 are disposed on and protrude from a surface of the flexible substrate layer 150A, such as an upper surface of the flexible substrate layer 150A (relative to the orientation seen in FIG. 4A). In some examples, the electrodes 160 are disposed on only one side of the substrate layer 150A, with electrical interconnections (such as traces) 161 that are connected and supply power thereto being routed on an opposite surface/side (i.e., a lower surface relative to the orientation seen in FIG. 4A) of the flexible substrate layer 150A. In some examples, electrical interconnections 161 can be disposed on both the upper and lower surfaces of the flexible substrate layer 150A.

With reference to FIG. 3, and as mentioned above, each flexible circuit 110, 150 can include the following components, with specific reference made to the flexible circuit 150. The flexible circuit 150 includes a base 151 comprising a soldering pad region 151A disposed at a proximal end thereof and a distal end 151B, a plurality of tines 152-156 extending from the base 151 along the longitudinal axis 61, a plurality of voids 159A-159F defined between the tines 152-156, and a connecting distal segment 158.

More specifically, the flexible substrate layer 150A and flexible circuit 150 include a central tine 154 that extends along the longitudinal axis 61 and offset tines 152, 153, 155, 156 that are offset from the longitudinal axis. The offset tines 152, 153, 155, 156 extend from the base 151 obliquely away from the longitudinal axis 61 and then generally along/parallel thereto. Multiple electrodes 160 are disposed on each tine 152-156. The electrodes 160 can be disposed along the tines 152-156 such that they are aligned relative to electrodes 160 on adjacent tines 152-156 along the longitudinal axis 60. In other examples, the electrodes 160 can be unaligned (i.e., staggered) relative to electrodes 160 on adjacent tines in a direction transverse to the longitudinal axis 61 such that they are arranged in an alternatingly aligned pattern from tine to tine.

The tines 152-156 include a first tine 152 extending from the base 151 along the longitudinal axis 61, a second tine 153 extending from the base 151 along the longitudinal axis 61, a third tine 154 extending from the base 151 along the longitudinal axis, a fourth tine 155 extending from the base 151 along the longitudinal axis 61, and a fifth tine 156 extending from the base 151 along the longitudinal axis 61. As seen in FIG. 3, the first through fifth tines 152-156 are consecutively arranged from left to right. The flexible substrate layer 150A and flexible circuit 150 further include a connecting distal segment 158 connecting distal ends of the first tine 152, the second tine 153, the third tine 154, the fourth tine 155, and the fifth tine 156.

Additionally, the tines include connecting outer segments 152A, 156A that do not include electrodes 160. Rather, these segments aid in defining the shape of the end effector 100 and provide reinforcement/protection to the segments of the flexible circuit 150 that carries electrical traces 161 and/or electrodes 160. The tines 152-156, connecting distal segment 158, and connecting outer segments collective define respective voids 159A-159F (i.e., an area not covered by any material) therebetween. This reduction of material can aid in facilitating the collapsing of the end effector 100 into the sheath 210 (FIG. 7) and/or insertion tool.

The flexible substrate layer 150A (and, as a whole, the flexible circuit 150) are divided into zones. As seen in FIG. 3, this example includes first and second zones - Zone A (the first zone) and Zone B (the second zone). Zone A includes the distal end 151B of the base 151 as well proximal portions of the tines 152-156. In some examples, Zone A can extend to a proximal-most end PE of the flexible circuit 150. Zone B includes distal portions of the tines 152-156, with Zone B and Zone A bordering/abutting one another. Zones A and B are sectioned such that the electrodes 160 are not disposed in Zone A but are in Zone B.

As mentioned above, one or more stiffening layers 140 are provided on a portion of the end effector 100 in order minimize kinking of the end effector 100 and/or to reduce strain on the electrical interconnections 161 in the region of the stiffening layer(s) 140. In some examples, a stiffening layer 140 is associated with both flexible circuits 110, 150. However, the stiffening layer 140 can also be provided with only one of the flexible circuits 110, 150 without departing from the spirit and scope of the present disclosure.

As seen in FIGs. 3 and 4A, the stiffening layer 140 is provided on the on the first side (i.e., the upper side of the substrate layer 150A relative to the orientation of FIG. 4A, the same side that the electrodes 160 are provided on) of the flexible substrate layer 150A and in Zone A of the flexible circuit 150. In some examples, the stiffening layer is provided only in Zone A and extends to a distal end thereof, such that the stiffening layer 140 terminates short of the electrodes 160 so that the electrodes 160 can conform to tissue. The first side, when the flexible circuit 150 is assembled with the rest of the components of the end effector 100, faces outwardly from the framework 120. This results in an asymmetrical stiffness of the flexible circuit 150, in Zone A, relative to the longitudinal axis 61 in the vertical direction V-V of the flexible circuit 150.

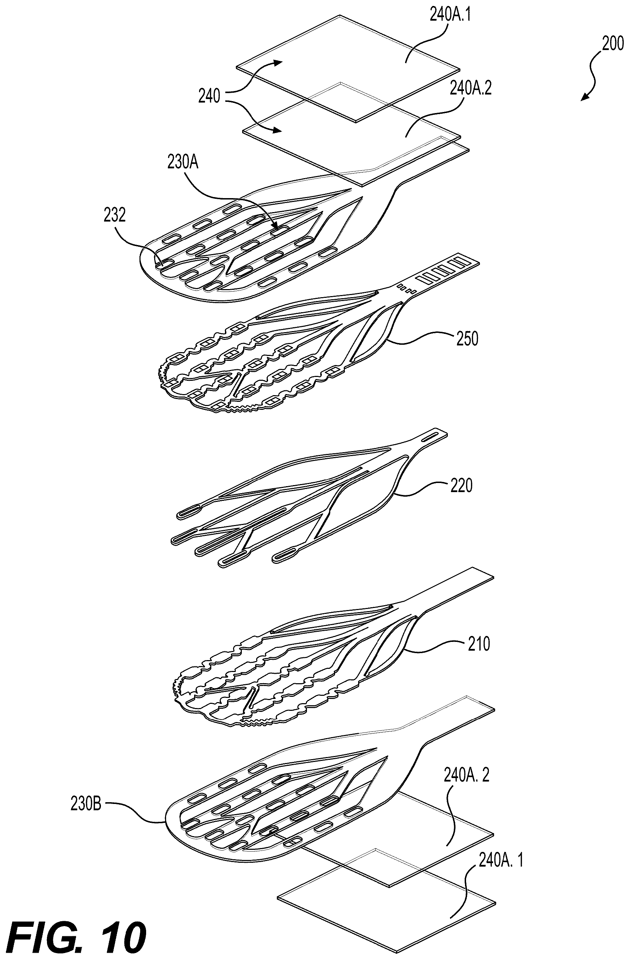

In the present example of FIGs. 3-4B, the stiffening layer 140 includes one or more flexible biocompatible material layers 140A and an optional connection layer 140B that connects the biocompatible material layer(s) 140A to the coverlay 150C. In the present example, a single biocompatible material layer 140A is used, but two or more layers 140A (e.g., as seen in FIG. 10) can also be used without departing from the spirit and scope of the present disclosure. In some examples, the stiffening layer 140 and the substrate layer 150A can form a monolithic structure. In some examples, the biocompatible material layer 140A comprises polyimide or TPU with a Shore A hardness of approximately 62 (e.g., in the range of 60-65 Shore A hardness). However, any appropriate material with a similar Shore A hardness can be used. In the present example, because of its higher Shore A hardness (as well as the increased overall thickness), the harder stiffening layer 140 (relative to the softer insulative material 130 with a lower Shore A hardness), when disposed on a portion of the flexible substrate layer 150A, gives that portion a greater flexural rigidity than that of other portions of the end effector 100 that are not provided with the stiffening layer.

In some examples, the optional connection layer 140B is an adhesive layer 140B. Of course, any appropriate assembly/connection process can be used. The stiffening layer 140 is provided on, in Zone A, the distal end 151B of the base 151 and all of the tines 152-156 such that the stiffening layer 140 comprises a base portion 151 overlaying the distal end 151B of the base 151 and tine portions 142-146 that overlay each portion of tines 152-156 that falls in Zone A.

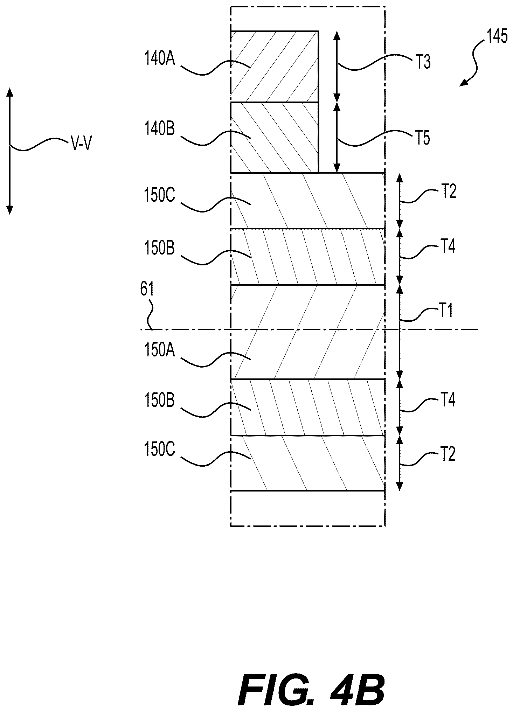

Making reference to the detail view of FIG. 4B, in the vertical direction V-V of the flexible circuit 150, each component of the flexible circuit 150 has a respective thickness. In particular, the flexible substrate layer 150A has a first thickness T1, the coverlays 150C on both sides of the flexible substrate layer 150A each have second thicknesses T2, the biocompatible material layer 140A of the stiffening layer 140 has a third thickness T3, the adhesive material layers 150B have a fourth thickness T4, and the connection layer 140B has a fifth thickness T5. In some examples, the first thickness T1 is approximately 25 microns, the second thickness T2 is approximately 12 microns, the third thickness T3 is approximately 25 microns, the fourth thickness T4 is approximately 12 microns, and the fifth thickness T5 is approximately 25 microns. However, it will be appreciated that any of T1-T5 can fall within the range of approximately 12-50 microns without departing from the spirit and scope of the present disclosure.

In contrast, Zone B, which extends along the longitudinal axis 61 from Zone A to the distal-most end DE of the flexible circuit 150, does not include the stiffening layer 140. This results in an overall thickness of Zone A (i.e., T1 + T2(x2) + T3 + T4(x2) + T5) of the flexible circuit 150 being greater than an overall thickness of Zone B (i.e., T1 + T2(x2) + T3) of the flexible circuit 150 and the flexural rigidity of Zone A being greater than the flexural rigidity of Zone B. In some examples, a maximum thickness of Zone A is at least 50% greater than a maximum thickness of Zone B (excluding potential protrusion of the electrodes 160). In some examples, the thickness in the Zone B is approximately 75 microns and the thickness in Zone A is approximately 125 microns.

As seen particularly in FIG. 4B, the stiffening layer 140 has a transition section 145 proximal to Zone B where the stiffening layer 140 terminates. In the example of FIGs. 4A-4B, this transition section 145 takes a substantially stepped profile (i.e., there is not a gradual reduction in material of the stiffening layer 140), and the thickness of the stiffening layer 40 is generally uniform.

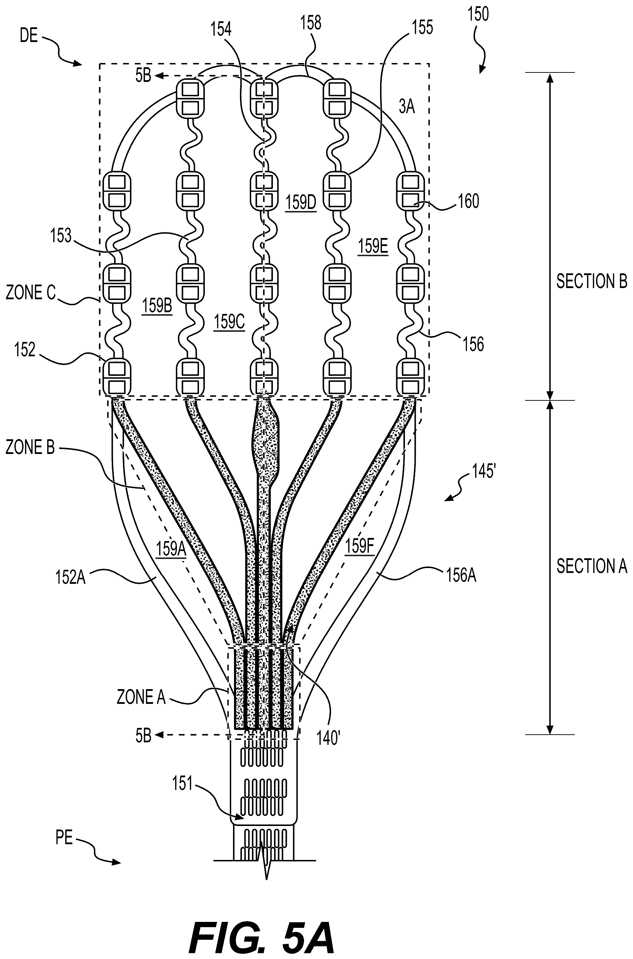

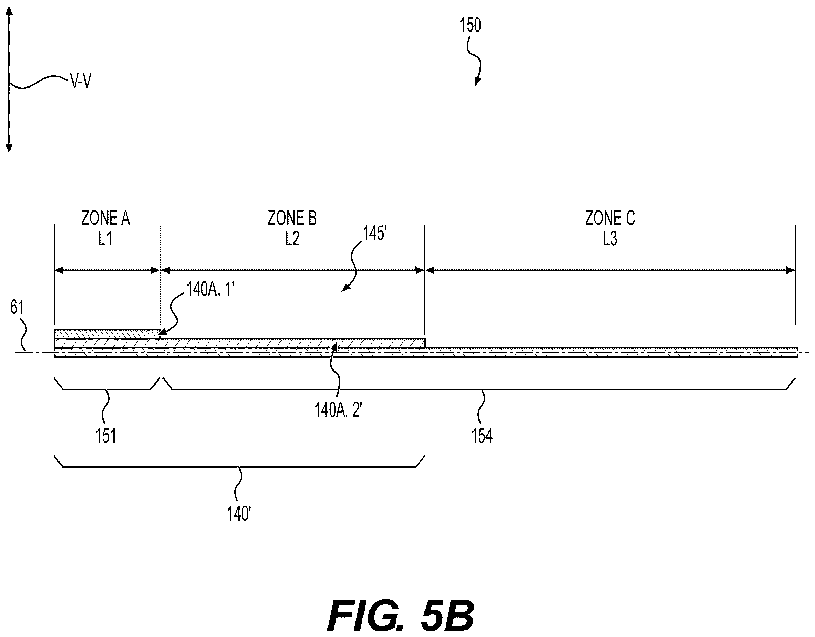

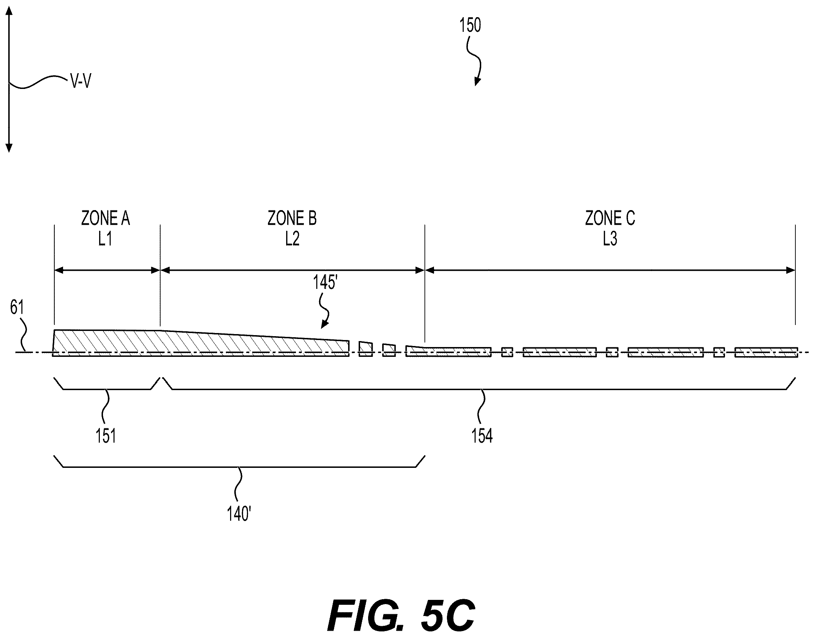

FIGs. 5A-5C depict an alternative configuration 140' (denoted by stippling in FIG. 5A) of the stiffening layer 140 where a gradual reduction in material/thickness is employed to achieve a stiffening layer that permits additional flexibility of the flexible circuit 150 at the distal end of the stiffening layer 140'. In this example, the stiffening layer 140' also extends in a first zone (Zones A and B, generally depicted as Section A) and does not extend in a second zone (Zone C, generally depicted also as Section B) that includes the electrodes 160. Rather than a uniform thickness in its entirety, the first zone is subdivided into Zones A and B, where the stiffening layer 140' spanning Zone A can have a uniform thickness and the stiffening layer 140' spanning Zone B can have a transition section 145'that is different in thickness and/or hardness relative to Zone A. In some examples, this can be achieved by the stiffening layer 140' being multi-layered using two or more sheets (discussed in greater detail with respect to FIG. 10). As seen in FIG. 5B, two sheets 140A.1', 140A.2' can be stacked in Zone A, with only a single sheet 140A.2' extending in Zone B. Of course, the degree of tapering in hardness can be further tuned by using more than two sheets that terminate at various locations along the transition section 145'. Alternatively, as seen in FIG. 5C the transition section 145' can have a tapered profile that gradually reduces in thickness from Zone A to Zone C. These configurations can be employed to achieve varying stiffnesses in the proximal section of the end effector as required by the design. In some examples, and as seen in FIG. 5B, the transition section 145'/Zone B can begin at the point where the tines 152-156 are defined and extend from the base 151.

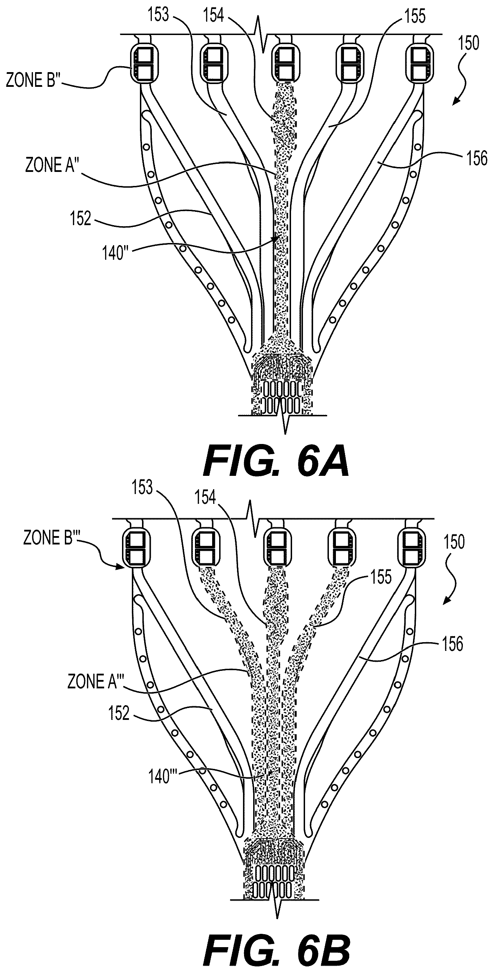

FIGs. 6A-6B depict other alternative configurations 140", 140‴ (denoted by stippling in FIGs. 6A and 6B) of the stiffening layer 140 consistent with the principles of the present disclosure. In these examples, the first zone and the stiffening layer is provided only on certain tines, rather than all of the tines. For example, as seen in FIG. 6A, the stiffening layer 140" can span Zone A", which is only over the central tine 154. Consequently, the offset tines 152, 153, 155, 156 do not have a stiffening layer, and only the central tine 154 is stiffened relative to a remainder (Zone B") of the flexible circuit 150. Similarly, as seen in FIG. 6B, another alternative stiffening layer 140‴ can span Zone A"', which is only over the three middle tines 153-155. Consequently, the outermost tines 152, 156 do not have a stiffening layer, and only middle tines 153-155 are stiffened relative to a remainder (Zone B") of the flexible circuit 150.

The present disclosure provides a medical probe assembly 200 as shown in FIG. 7 which can include a tubular member 230 extending along a longitudinal axis 60 and configured to deliver any of the end effectors 100 previously discussed to and out of a sheath 210. A physician 24 can manipulate the medical probe 200 with handle 220. Appropriate examples for catheter assembly 200 and its subcomponents such as handle 220, sheath 210, tubular member 230, and others not mentioned herein are described in US Patent publication No. 2021/0369339, which is incorporated herein by reference.

Further to the above-described examples, and with reference to FIG. 8, a method 800 of manufacturing an end effector for a medical device can include the following. A flexible substrate layer of a flexible circuit is formed 802. The flexible substrate layer includes a first surface disposed on a first side of the flexible substrate layer, a second surface disposed on a second side of the flexible substrate layer, a plurality of tines, a first zone comprising a portion of at least one of the tines of the plurality of tines, and a second zone comprising another portion of the plurality of tines. A plurality of electrodes is formed 804 on the first side. A stiffening layer is formed 806 on the first side of the flexible substrate layer and in the first zone of the flexible circuit such that (i) a first thickness of the first zone of the flexible circuit is greater than a second thickness of the second zone of the flexible circuit, the thickness being measured along a vertical axis, and (ii) a first flexural rigidity of the first zone is greater than a second flexural rigidity of the second zone.

In some examples, the forming 804 the electrodes on the first surface includes forming the electrodes in the second zone of the flexible substrate. In some examples, forming 806 the stiffening layer on the first side of the flexible substrate layer includes adhering a flexible biocompatible material layer to the flexible substrate. In some examples, forming 802 the flexible substrate includes adhering a coverlay to the first surface of the flexible substrate. In some examples, forming 806 the stiffening layer on the first side of the flexible substrate layer includes adhering the stiffening layer to the coverlay. In some examples, forming 802 the flexible substrate layer and forming 806 the stiffening layer steps includes molding a flexible biocompatible material to form the flexible substrate and the stiffening layer.

Other methods can include, but are not limited to, providing a polyimide sheet that is pre-laser cut with adhesive and placed on the main flexible circuit. Alternatively, a photo imageable coverlay (stiffener) can be provided that solidifies when exposed to ultraviolet light (rather than a pre-laser cut process in the previously described alternative method).

Turning now to FIGs. 9-10, an alternative configuration 200 of end effector 100 is depicted. In this example, an insulative material 230 (equivalent to the previously described insulative material 130) is provided as first and second sheets 230A, 230B that are moved from between the flexible circuits 210, 250 (equivalent to the previously described flexible circuits 110, 150) and framework 220 (equivalent to the previously described framework 120) to being the outermost portions of the end effector 200 along the vertical axis 62. As seen in FIG. 9, the insulative material 230A, 230B includes voids 232 that expose the electrodes of the flexible circuits 210, 250. In this example, since the insulative material 230A, 230B is, as part of the manufacturing process, stacked on the outer portions of the end effector (i.e., separated from the framework 220 by the flexible circuits 210, 250 along the vertical axis 62), little to no insulative material may be disposed between the flexible circuits 210, 250 and framework 220 when the insulative material 230A, 230B is heat formed around at least a portion of the first flexible circuit 250, the second flexible circuit 210, and the framework 220. This configuration of insulative material 230A, 230B can be applied to any of the preceding examples without departing from the spirit and scope of the present disclosures.

As seen in FIG. 10, and/or in conjunction with any of the previously described examples as mentioned above (e.g., with respect to the description of FIGs. 5A-5B), a multi-layered stiffening layer 240 can be provided that is disposed on the insulative material 230A, 230B. As seen, stiffening layers 240 can be provided on both the first insulative sheet 230A and the second insulative sheet 230B. The stiffening layers can each be configured as two or more sheets that overlay one another and are fused together along with the insulative material 230. In this example, each stiffening layer includes two biocompatible material layers 240A.1, 240A.2 stacked in the aforementioned Zone A of FIG. 3. These biocompatible material layers 240A.1, 240A.2 can comprise polyimide or TPU with a Shore A hardness of approximately 62 (e.g., in the range of 60-65 Shore A hardness), with the insulative material 230A, 230B having a Shore A hardness of approximately 52 (e.g., a Shore A hardness in the range of 50-55). Besides the arrangement of the insulative material and the multi-layering of the stiffening layer 240, the features of the previously described examples can otherwise be incorporated herein. Therefore, for the purposes of brevity, further description of the present example is omitted/unnecessary.

The disclosed technology described herein can be further understood according to the following clauses:
Clause 1. A flexible circuit for an end effector of a medical probe, the flexible circuit comprising: a flexible substrate layer extending along a longitudinal axis, the flexible substrate layer comprising: a first side and a second side along a vertical axis that is orthogonal to the longitudinal axis; a plurality of tines extending along the longitudinal axis; a first zone comprising a portion of at least one of the tines of the plurality of tines; and a second zone comprising another portion of the plurality of tines; a plurality of electrodes disposed on each of the plurality of tines of the flexible substrate layer; and a stiffening layer disposed on the first side of the flexible substrate layer and in the first zone of the flexible circuit such that (i) a first thickness of the first zone of the flexible circuit is greater than a second thickness of the second zone of the flexible circuit, the thickness being measured along the vertical axis, and (ii) a first flexural rigidity of the first zone is greater than a second flexural rigidity of the second zone.
Clause 2. The flexible circuit of clause 1, the plurality of electrodes being disposed on the second zone of the flexible circuit.
Clause 3. The flexible circuit of any one of clauses 1-2, the second zone extending from the first zone to a distalmost end of the flexible circuit.
Clause 4. The flexible circuit of any one of clauses 1-3, the flexible circuit being asymmetrically stiff relative to the longitudinal axis in a vertical direction of the flexible circuit.
Clause 5. The flexible circuit of clause 4, the flexible circuit being asymmetrically stiff in the first zone.
Clause 6. The flexible circuit of any one of clauses 1-5, the plurality of electrodes being disposed on the first side of the flexible substrate layer.
Clause 7. The flexible circuit of any one of clauses 1-6, the stiffening layer terminating at a distal end of the first zone, and the plurality of electrodes being disposed in the second zone.
Clause 8. The flexible circuit of any one of clauses 1-7, the stiffening layer comprising a transition section proximal to the second zone, the transition section comprising one of a stepped profile or a tapered profile.
Clause 9. The flexible circuit of any one of clauses 1-9, the flexible substrate comprising a base, and the plurality of tines comprising: a first tine extending from the base along the longitudinal axis; a second tine extending from the base along the longitudinal axis; a third tine extending from the base along the longitudinal axis; a fourth tine extending from the base along the longitudinal axis; and a fifth tine extending from the base along the longitudinal axis.
Clause 10. The flexible circuit of clause 9, the stiffening layer being disposed on the base and the third tine.
Clause 11. The flexible circuit of clause 9, the stiffening layer being disposed on the base, the second tine, the third tine, and the fourth tine.
Clause 12. The flexible circuit of clause 9, the stiffening layer being disposed on the base, the first tine, the second tine, the third tine, the fourth tine, and the fifth tine.
Clause 13. The flexible circuit of any one of clauses 9-12, the third tine aligning with the longitudinal axis, and the first tine, the second tine, the fourth tine, and the fifth tine being offset from the longitudinal axis.
Clause 14. The flexible circuit of any one of clauses 9-13, two or more electrodes of the plurality of electrodes being disposed on each tine.
Clause 15. The flexible circuit of any one of clauses 9-14, each tine extending in the first zone and the second zone.
Clause 16. The flexible circuit of any one of clauses 9-15, the flexible substrate comprising a plurality of voids defined between the tines.
Clause 17. The flexible circuit of any one of clauses 1-16, the first thickness being at least fifty percent greater than the second thickness.
Clause 18. The flexible circuit of any one of clauses 1-17, further comprising a coverlay disposed between the flexible substrate and the stiffening layer, the stiffening layer being disposed on the coverlay.
Clause 19. The flexible circuit of clause 18, the stiffening layer comprising an adhesive layer that connects the stiffening layer to the coverlay.
Clause 20. The flexible circuit of any one of clauses 1-17, the stiffening layer and the flexible substrate layer forming a monolithic structure.
Clause 21. The flexible circuit of any one of clauses 1-20, the stiffening layer comprising a flexible biocompatible material layer comprising a Shore A hardness of approximately 62.
Clause 22. The flexible circuit of any one of clauses 1-20, the stiffening layer comprising a first flexible biocompatible material layer and a second flexible biocompatible material layer stacked on one another.
Clause 23. The flexible circuit of clause 22, the stiffening layer comprising a third flexible biocompatible material layer stacked on the first and second biocompatible material layers.
Clause 24. The flexible circuit of any one of clauses 1-23, the stiffening layer comprising polyimide.
Clause 25. An end effector for a medical probe, the end effector comprising: an insulative material; a framework disposed in the insulative material, the framework being approximately planar along a first longitudinal axis; and a first flexible circuit disposed in the insulative material such that the first flexible circuit is spaced apart from the framework along a vertical axis that is orthogonal to the first longitudinal axis, the first flexible circuit comprising: a first flexible substrate layer extending along a second longitudinal axis parallel to the first longitudinal axis, the first flexible substrate layer comprising: a first side and a second side along a vertical axis that is orthogonal to the longitudinal axis; a plurality of tines extending along the longitudinal axis; a first zone comprising a portion of at least one of the tines of the plurality of tines; and a second zone comprising another portion of the plurality of tines; a plurality of electrodes disposed on each of the plurality of tines of the flexible substrate layer; and a stiffening layer disposed on the first side of the flexible substrate layer and in the first zone of the flexible circuit such that (i) a first thickness of the first zone of the flexible circuit is greater than a second thickness of the second zone of the flexible circuit, the thickness being measured along the vertical axis, and (ii) a first flexural rigidity of the first zone is greater than a second flexural rigidity of the second zone.
Clause 26. The end effector of clause 25, the first side of the first flexible substrate layer facing away from the framework.
Clause 27. The end effector of any one of clauses 25-26, the first stiffening layer extending, along the vertical axis, from the first flexible substrate layer away from the framework.
Clause 28. The end effector of any one of clauses 25-27, further comprising: a second flexible circuit disposed in the insulative material such that the second flexible circuit is spaced apart from the framework and the first flexible circuit along the vertical axis.
Clause 29. The end effector of clause 28, the second flexible circuit comprising: a second flexible substrate layer, the flexible substrate layer having a first side and a second side along the vertical axis; and a second stiffening layer disposed on the first side of the second flexible substrate layer and in a first zone of the second flexible circuit such that (i) a first thickness of the first zone of the second flexible circuit is greater than a second thickness of a second zone of the second flexible circuit, the thickness being measured along the vertical axis, and (ii) a first flexural rigidity of the first zone of the second flexible circuit is greater than a second flexural rigidity of the second zone of the second flexible circuit.
Clause 30. The end effector of clause 29, the second stiffening layer extending, along the vertical axis, from the second flexible substrate layer away from the framework.
Clause 31. An end effector for a medical probe, the end effector comprising: an insulative material; a framework disposed in the insulative material, the framework being approximately planar along a first longitudinal axis; and a first flexible circuit disposed in the insulative material, the first flexible circuit comprising: a first flexible substrate layer extending along a second longitudinal axis parallel to the first longitudinal axis, the first flexible substrate layer comprising: a first side and a second side along a vertical axis that is orthogonal to the first longitudinal axis; a plurality of tines extending along the longitudinal axis; a first zone comprising a portion of at least one of the tines of the plurality of tines; and a second zone comprising another portion of the plurality of tines; a plurality of electrodes disposed on each of the plurality of tines of the flexible substrate layer; and a stiffening layer disposed on the insulative material on the first side of the first flexible substrate layer and in the first zone of the flexible circuit such that (i) a first thickness of the first zone of the flexible circuit is greater than a second thickness of the second zone of the flexible circuit, the thickness being measured along the vertical axis, and (ii) a first flexural rigidity of the first zone is greater than a second flexural rigidity of the second zone, the stiffening layer comprising a plurality of biocompatible material layers stacked on one another.
Clause 32. The end effector of clause 31, further comprising a second flexible circuit, the insulative material comprising a first insulative sheet and a second insulative sheet, the first and second insulative sheets each respectively being spaced from framework, by the first flexible circuit and the second flexible circuit, along a vertical axis that is orthogonal to the vertical axis.
   insulative material such that the first flexible circuit is spaced apart from the framework along a vertical axis that is orthogonal to the vertical axis
Clause 33. A method comprising: forming a flexible substrate layer of a flexible circuit, the flexible substrate layer comprising: a first surface disposed on a first side of the flexible substrate layer, a second surface disposed on a second side of the flexible substrate layer, a plurality of tines, a first zone comprising a portion of at least one of the tines of the plurality of tines, and a second zone comprising another portion of the plurality of tines; forming a plurality of electrodes on the first surface; and forming a stiffening layer on the first side of the flexible substrate layer and in the first zone of the flexible circuit such that (i) a first thickness of the first zone of the flexible circuit is greater than a second thickness of the second zone of the flexible circuit, the thickness being measured along a vertical axis, and (ii) a first flexural rigidity of the first zone is greater than a second flexural rigidity of the second zone.
Clause 34. The method of clause 33, the forming the electrode on the first surface comprising: forming the electrode on the first surface in the second zone of the flexible substrate.
Clause 35. The method of any one of clauses 33-34, the forming the stiffening layer on the first side of the flexible substrate layer comprising: adhering a flexible biocompatible material layer to the flexible substrate.
Clause 36. The method of any one of clauses 33-35, the forming the flexible substrate comprising: adhering a coverlay to the first surface of the flexible substrate.
Clause 37. The method of clause 36, the forming the stiffening layer on the first side of the flexible substrate layer comprising: adhering the stiffening layer to the coverlay.
Clause 38. The method of any one of clauses 33-34, the forming the flexible substrate layer and the forming the stiffening layer steps comprise: molding a flexible biocompatible material to form the flexible substrate and the stiffening layer.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. A flexible circuit for an end effector of a medical probe, the flexible circuit comprising:
a flexible substrate layer extending along a longitudinal axis, the flexible substrate layer comprising:
a first side and a second side along a vertical axis that is orthogonal to the longitudinal axis;
a plurality of tines extending along the longitudinal axis;
a first zone comprising a portion of at least one of the tines of the plurality of tines; and
a second zone comprising another portion of the plurality of tines;
a plurality of electrodes disposed on each of the plurality of tines of the flexible substrate layer; and
a stiffening layer disposed on the first side of the flexible substrate layer and in the first zone of the flexible circuit such that (i) a first thickness of the first zone of the flexible circuit is greater than a second thickness of the second zone of the flexible circuit, the thickness being measured along the vertical axis, and (ii) a first flexural rigidity of the first zone is greater than a second flexural rigidity of the second zone.

2. The flexible circuit of claim 1, the plurality of electrodes being disposed on the second zone of the flexible circuit.

3. The flexible circuit of claim 1 or claim 2, the second zone extending from the first zone to a distalmost end of the flexible circuit.

4. The flexible circuit of claim 1 to 3, the flexible circuit being asymmetrically stiff relative to the longitudinal axis in a vertical direction of the flexible circuit, optionally the flexible circuit being asymmetrically stiff in the first zone.

5. The flexible circuit of claim 1 to 4, the plurality of electrodes being disposed on the first side of the flexible substrate layer.

6. The flexible circuit of claim 1 to 5, the stiffening layer terminating at a distal end of the first zone, and the plurality of electrodes being disposed in the second zone.

7. The flexible circuit of claim 1 to 6, the stiffening layer comprising a transition section proximal to the second zone, the transition section comprising one of a stepped profile or a tapered profile.

8. The flexible circuit of any one of claim 1 to 7, the flexible substrate comprising a base, and the plurality of tines comprising:
a first tine extending from the base along the longitudinal axis;
a second tine extending from the base along the longitudinal axis;
a third tine extending from the base along the longitudinal axis;
a fourth tine extending from the base along the longitudinal axis; and
a fifth tine extending from the base along the longitudinal axis.

9. The flexible circuit of claim 8, the stiffening layer being disposed on (i) the base and the third tine, (ii) the base, the second tine, the third tine, and the fourth tine, or (iii) the base, the first tine, the second tine, the third tine, the fourth tine, and the fifth tine.

10. The flexible circuit of claim 8 or claim 9, the third tine aligning with the longitudinal axis, and the first tine, the second tine, the fourth tine, and the fifth tine being offset from the longitudinal axis.

11. The flexible circuit of claim 1 to 10, the first thickness being at least fifty percent greater than the second thickness.

12. The flexible circuit claim 1 to 11, further comprising a coverlay disposed between the flexible substrate and the stiffening layer, the stiffening layer being disposed on the coverlay.

13. An end effector for a medical probe, the end effector comprising:
an insulative material;
a framework disposed in the insulative material, the framework being approximately planar along a first longitudinal axis; and
a first flexible circuit disposed in the insulative material such that the first flexible circuit is spaced apart from the framework along a vertical axis that is orthogonal to the first longitudinal axis, the first flexible circuit comprising:
a first flexible substrate layer extending along a second longitudinal axis parallel to the first longitudinal axis, the first flexible substrate layer comprising:
a first side and a second side along a vertical axis that is orthogonal to the longitudinal axis;
a plurality of tines extending along the longitudinal axis;
a first zone comprising a portion of at least one of the tines of the plurality of tines; and
a second zone comprising another portion of the plurality of tines;
a plurality of electrodes disposed on each of the plurality of tines of the flexible substrate layer; and
a stiffening layer disposed on the first side of the flexible substrate layer and in the first zone of the flexible circuit such that (i) a first thickness of the first zone of the flexible circuit is greater than a second thickness of the second zone of the flexible circuit, the thickness being measured along the vertical axis, and (ii) a first flexural rigidity of the first zone is greater than a second flexural rigidity of the second zone.

14. The end effector of claim 13, (i) the first side of the first flexible substrate layer facing away from the framework and/or (ii) the first stiffening layer extending, along the vertical axis, from the first flexible substrate layer away from the framework, and/or (iii) further comprising a second flexible circuit disposed in the insulative material such that the second flexible circuit is spaced apart from the framework and the first flexible circuit along the vertical axis.

15. A method comprising:
forming a flexible substrate layer of a flexible circuit, the flexible substrate layer comprising:
a first surface disposed on a first side of the flexible substrate layer,
a second surface disposed on a second side of the flexible substrate layer,
a plurality of tines,
a first zone comprising a portion of at least one of the tines of the plurality of tines, and
a second zone comprising another portion of the plurality of tines;
forming a plurality of electrodes on the first surface; and
forming a stiffening layer on the first side of the flexible substrate layer and in the first zone of the flexible circuit such that (i) a first thickness of the first zone of the flexible circuit is greater than a second thickness of the second zone of the flexible circuit, the thickness being measured along a vertical axis, and (ii) a first flexural rigidity of the first zone is greater than a second flexural rigidity of the second zone.
